Europäisches Patentamt

(19) European Patent Office

Office européen des brevets

(11) Veröffentlichungsnummer: **0 164 329**
**B1**

(12) **EUROPÄISCHE PATENTSCHRIFT**

(45) Veröffentlichungstag der Patentschrift:
25.01.89

(51) Int. Cl.⁴: **A 61 K 6/04,** C 22 C 19/07,
C 22 C 30/00

(21) Anmeldenummer: 85890066.3

(22) Anmeldetag: 03.04.85

(54) Dentallegierung.

(30) Priorität: 06.04.84 AT 1167/84

(43) Veröffentlichungstag der Anmeldung:
11.12.85 Patentblatt 85/50

(45) Bekanntmachung des Hinweises auf die Patenterteilung:
25.01.89 Patentblatt 89/4

(84) Benannte Vertragsstaaten:
CH DE FR GB IT LI SE

(56) Entgegenhaltungen:
EP-A-0 041 938
US-A-4 263 045

ZWR, Das Deutsche Zahnärzteblatt, August 1985,
Dr.A. Hüthig Verlag Gmbh, Heidelberg, Seiten 618-
622
Sonderdruck aus ZWR 5/86, Seiten 544-550

(73) Patentinhaber: VEREINIGTE EDELSTAHLWERKE
AKTIENGESELLSCHAFT (VEW), Elisabethstrasse
12, A-1010 Wien (AT)

(72) Erfinder: Kulmburg, Alfred, Dr., Richard Wagner-
Gasse 37, A-8605 Kapfenberg (AT)

**Beschreibung**

Die Erfindung bezieht sich auf die Verwendung einer Kobaltlegierung als Werkstoff für mit Dentalkeramik zu verblendenden Kronen, Brücken od. dgl

Bei Legierungen, die zur Verwendung im menschlichen Körper bestimmt sind, ist eine Grundvoraussetzung die physiologische Unbedenklichkeit. Neben der an sich naheliegenden Tatsache, daß sich die Legierungen im menschlichen Körper nicht auflösen sollen, besteht beim Einsatz der Legierungen in der Mundhöhlung eine Reihe von weiteren Auflagen. Diese Legierungen müssen gegenüber den sehr stark korrosiven Beanspruchungen, z. B. extrem schwankender pH-Wert in der Mundhöhlung mit gleichzeitig unterschiedlichen Konzentrationen an korrosionsfördernden Ionen, z. B. Chlorionen, auch noch Eigenschaften aufweisen, welche im Laufe der Herstellung von Zahnersatz, Brücken, od dgl. von Bedeutung sind. Aus optischen und auch aus Gründen der Haltbarkeit werden Zahnprothesen mit einer Keramik verkleidet, wobei diese Keramik durch Brennvorgänge auf die Metalloberfläche aufgebracht wird. Beim Aufbrennen der Keramik muß berücksichtigt werden, daß die Temperatur ca. um 1000°C angehoben wird, womit die Unterschiede in thermischen Ausdehnungskoeffizienten nicht sehr hoch sein dürfen und weiters soll, wenn bereits Unterschiede bestehen, die thermische Ausdehnung der Legierungskomponente geringer sein, als jene der keramischen, damit der keramische Anteil nach Abkühlen des Zahnersatzes nicht unter Zug-, sondern unter Druckspannung steht, da Keramik nur eine geringe Zugfestiget, jedoch eine hohe Druckfestigkeit, aufweist.

Weiters besteht des öfteren der Wunsch, daß die Keramik nicht durch einen Brandvorgang, sondern durch mehrere Brennvorgänge aufgebracht wird, und das Verhalten bei diesen einzelnen Brennvorgängen soll dem eingangs dargelegten Verhalten entsprechen.

Die Aufgabe der vorliegenden Erfindung besteht nun darin, die Verwendung einer Legierung für keramisch zu verblendende dentale Prothetik in Vorschlag zu bringen, die in ihrem thermischen Verhalten besonders gut geeignet ist, wobei auch mehrmalige Wärmebehandlungen keine nachträgliche Veränderung des linearen Wärmeausdehnungskoeffizienten bedingen. Die Erfindung besteht in der Verwendung einer Kobaltlegierung mit in Gew.-% Kohlenstoff 0 bis 0,4, vorzugsweise 0,05 bis 0,3, Silizium 0,1 bis 5,0, vorzugsweise 0,1 bis 3,0, Mangan 0,01 bis 8,0, vorzugsweise 0,3 bis 3,0, Chrom 25 bis 35, vorzugsweise 27 bis 33, Molybdän 1,0 bis 8,0, vorzugsweise 4 bis 7, Niob 0,1 bis 5, vorzugsweise 0,5 bis 3, Nickel 0 bis 0,3, Eisen 0 bis 1,0 und Kupfer 0,1 bis 5,0, vorzugsweise 0,5 bis 2,0, Rest Kobalt und herstellungsbedingte Verunreinigungen für keramisch zu verblendende Metallgerüste bei der dentalen Prothetik.

Eine Legierung mit der erfindungsgemäßen Zusammensetzung weist den für keramisch zu verblendende Dentalgerüste erwünschten Ausdehnungskoeffizienten auf, wobei gleichzeitig die erforderlichen mechanischen Werte, insbesondere Bruchdehnung und Härte ebenfalls erfüllt werden. Völlig überraschend war jedoch, daß diese Legierung eine hohe Maßbeständigkeit auch nach mehreren thermischen Beanspruchungen aufweist. Diese hohen thermischen Beanspruchungen treten nicht nur bei dem mehrmaligen Brand der Keramikschichte auf, sondern werden auch bei Anpassung der Prothese, z. B. an den Gegenbiß, verursacht, da der Zahnarzt durch mechanischen Abtrag der Keramikschichte ein Angleichen an den Gegenbiß durchführt, wodurch es zu extremen lokalen thermischen Beanspruchungen kommen kann. Tritt dann eine irreversible Maßänderung ein, kann es zur vorzeitigen Zerstörung der Keramik, bzw. z. B. durch Bildung von Haarrissen und Eindringen von Farbstoff zu einer unschönen Verfärbung der Keramik kommen.

Im folgenden wird die Erfindung anhand der Beispiele näher erläutert.

**Beispiele:**

Bei dem Aufbrennen von keramischen Stoffen z. B. 80 Gew.-% Feldspat, 18 Gew.-% Quarz und 2 Gew.-% Borfritte auf die zu verblendenden Gerüste wird die Probe ein- oder mehrmals auf Temperaturen zwischen 950 und 1000°C erhitzt, wobei diese Erhitzung in der Regel 10 Minuten beträgt und dazwischen erfolgt eine Abkühlung auf Raumtemperatur, um beispielsweise die zu brennende Masse aufzubringen. Um einen dreifachen Brand zu simulieren, und gleichzeitig die Dimensionsänderungen feststellen zu können, wurden verschiedene Probekörper einer Zusammensetzung gemäß Tabelle mit einen Durchmesser von 10 mm und einer Höhe von 10 mm im Vakuum zuerst auf 1000°C erhitzt, zehn Minuten auf dieser Temperatur gehalten, dann schnell auf Raumtemperatur abgekühlt. Dieser Vorgang wurde dreimal wiederholt. Es konnten keine Veränderungen des Durchmessers sowie der Höhe sowohl nach dem ersten, dem zweiten als auch dem dritten Brand festgestellt werden.

Wie der Tabelle zu entnehmen, bewegt sich der thermische Ausdehnungskoeffizient zwischen 13,7 und 15,5 x $10^{-6}$°C$^{-1}$ und liegt damit innerhalb der erwünschten Grenzen für die thermische Ausdehnung, um die aufzubringende Keramik unter einer erwünschten Druckspannung und nicht einer Zugspannung zu halten. Die Härte zwischen 290 und 410 HV 10, entspricht ebenfalls den Erfordernissen, wobei gleichzeitig Dehngrenze und Bruchdehnung eine entsprechende mechanische Bearbeitung erlauben.

| | | chem. Zusammensetzung | | | | | | | | | Eigenschaften | | |
| | | C | Si | Mn | Cr | Mo | Ni | Cu | Nb | Co | Härte HV10 | Bruch-Dehngrenzedehnung Rp0,2 N/mm² | % | Ausdehnungs-koeff.20 - 500°C 10⁻⁶°C⁻¹ |
|---|---|---|---|---|---|---|---|---|---|---|---|---|---|---|
| | 1 | 0,25 | 0,45 | 0,52 | 26,52 | 3,81 | 0,12 | 0,12 | 0,20 | Rest | 330 | 600 | 9 | 15,0 |
| | 2 | 0,38 | 0,64 | 6,80 | 31,12 | 5,12 | 0,16 | 0,32 | 0,12 | Rest | 300 | 585 | 10 | 13,7 |
| | 3 | 0,12 | 0,52 | 0,05 | 30,21 | 5,46 | 0,08 | 0,57 | 1,02 | Rest | 315 | 580 | 9 | 14,5 |
| | 4 | 0,02 | 0,61 | 0,18 | 32,12 | 4,96 | 0,05 | 0,28 | 3,80 | Rest | 290 | 560 | 11 | 14,8 |
| | 5 | 0,37 | 0,43 | 0,12 | 30,32 | 5,38 | 0,09 | 0,12 | 0,32 | Rest | 335 | 610 | 8 | 14,8 |
| | 6 | 0,39 | 2,80 | 0,20 | 29,82 | 5,08 | 0,11 | 0,50 | 0,21 | Rest | 410 | 680 | 5 | 15,5 |
| | 7 | 0,33 | 0,31 | 0,09 | 28,96 | 5,81 | 0,08 | 4,12 | 0,61 | Rest | 335 | 590 | 8 | 14,8 |
| | 8 | 0,21 | 1,06 | 0,51 | 34,79 | 5,27 | 0,07 | 0,23 | 0,16 | Rest | 370 | 630 | 6 | 13,9 |
| | 9 | 0,26 | 0,42 | 0,65 | 30,78 | 6,86 | 0,14 | 0,12 | 0,41 | Rest | 340 | 600 | 8 | 15,4 |

## Patentansprüche

1. Verwendung einer Kobaltlegierung mit in Gew.-% Kohlenstoff 0 bis 0,4, Silizium 0,1 bis 5,0, Mangan 0,01 bis 8,0, Chrom 25 bis 35, Molybdän 1,0 bis 8,0, Niob 0,1 bis 5, Nickel 0 bis 0,3, Eisen 0 bis 1,0 und Kupfer 0,1 bis 5,0, Rest Kobalt und herstellungsbedingte Verunreinigungen für keramisch zu verblendende Metallgerüste bei der dentalen Prothetik.

2. Verwendung einer Kobaltlegierung nach Anspruch 1 mit in Gew.-% Kohlenstoff 0,05 bis 0,3, Silizium 0,1 bis 3,0, Mangan 0,3 bis 3,0, Chrom 27 bis 33, Molybdän 4 bis 7, Niob 0,5 bis 3 und Kupfer 0,5 bis 2,0, Rest Kobalt und herstellungsbedingte Verunreinigungen für keramisch zu verblendende Metallgerüste bei der dentalen Prothetik.

## Claims

1. The use of a cobalt alloy with in percentages by weight 0 to 0.4 carbon, 0.1 To 5.0 silicon, 0.01 to 8.0 manganese, 25 to 35 chromium, 1.0 to 8.0 molybdenum, 0.1 to 5 niobium, 0 to 0.3 nickel, 0 to 1.0 iron and 0.1 to 5.0 copper, the remainder cobalt and impurities determined by the production, for metal frames to be coated by ceramic material in dental prosthetics.

2. The use of a cobalt alloy according to claim 1 with in percentages by weight 0.05 to 0.3 carbon, 0.1 to 3.0 silicon, 0.3 to 3.0 manganese, 27 to 33 chromium, 4 to 7 molybdenum, 0.5 to 3 niobium, and 0.5 to 2.0 copper, the remainder cobalt and impurities determined by the production, for metal frames to be coated by ceramic material in dental prosthetics.

## Revendications

1. Utilisation d'un alliage de cobalt contenant, en % en poids: carbone 0 à 0,4, silicium 0,1 à 5,0, manganèse 0,01 à 8,0, chrome 25 à 35, molybdène 1,0 à 8,0, niobium 0,1 à 5, nickel 0 à 0,3, fer 0 à 1,0 et cuivre 0,1 à 5,0, le reste étant constitué de cobalt et des impuretés découlant de la fabrication, pour des structures métalliques à revêtement céramique pour prothèse dentaire.

2. Utilisation d'un alliage de cobalt selon la revendication 1, avec, en % en poids carbone 0,05 à 0,3, silicium 0,1 à 3,0, manganèse 0,3 à 3,0, chrome 27 à 33, molybdène 4 à 7, niobium 0,5 à 3 et cuivre 0,5 à 2,0, le reste étant constitué de cobalt et des impuretés découlant de la fabrication, pour des structures métalliques à revêtement céramique pour prothèse dentaire.